Europäisches Patentamt

European Patent Office

Office européen des brevets

(11) Veröffentlichungsnummer: **0 275 444**
**A1**

(19)

## (12) EUROPÄISCHE PATENTANMELDUNG

(21) Anmeldenummer: **87118052.7**

(22) Anmeldetag: **07.12.87**

(51) Int. Cl.⁴: **A61K 31/505** , A61K 9/52

(30) Priorität: **09.12.86 CH 4894/86**

(43) Veröffentlichungstag der Anmeldung:
**27.07.88 Patentblatt 88/30**

(84) Benannte Vertragsstaaten:
**AT BE CH DE ES FR GB GR IT LI LU NL SE**

(71) Anmelder: **BYK GULDEN LOMBERG CHEMISCHE FABRIK GMBH**
**Postfach 6500 Byk-Gulden-Strasse 2**
**D-7750 Konstanz(DE)**

(72) Erfinder: **Benedikt, Gerald**
**Wintersberg Strasse 1**
**D-7750 Konstanz 19(DE)**
Erfinder: **Dietrich, Rango, Dr.**
**Im Tiergarten 16**
**D-7750 Konstanz 16(DE)**

(54) **Arzneiform für Urapidil.**

(57) Es wird eine Retardpellet-Arzneiform für Urapidil beschrieben, die neben Urapidil einen Überschuß einer pharmakologisch annehmbaren aliphatischen Dicarbonsäure enthält und mit einem magensaftresistenten Überzug versehen ist. Die neue Arzneiform erlaubt die täglich einmalige Verabreichung von Urapidil und zeichnet sich durch eine gute Bioverfügbarkeit aus.

EP 0 275 444 A1

## Arzneiform für Urapidil

### Technisches Gebiet

Die Erfindung bezieht sich auf eine Retardpellet-Arzneiform enthaltend Urapidil und eine pharmakologisch annehmbare aliphatische Dicarbonsäure.

### Stand der Technik

Der Wirkstoff 6-({3-[4-(o-Methoxyphenyl)-1-piperazinyl]propyl}amino)-1,3-dimethyluracil (INN: Urapidil) ist ein bekannter Wirkstoff zur Therapie von Bluthochdruck. Der pKa-Wert von Urapidil beträgt 7,10.

Urapidil weist eine interindividuell stark unterschiedliche Pharmakokinetik und eine verhältnismäßig kurze Eliminationshalbwertzeit von 2,7 Stunden auf. Es bereitet deshalb große galenische Schwierigkeiten, eine Zubereitungsform für die chronische orale Therapie zu schaffen, die stets wirksame Blutspiegel gewährleistet, aber Blutspiegelspitzen, die zu unerwünschten Nebenwirkungen, wie z.B. Orthostase führen, vermeidet.

Da die Hypertonie dem Patienten oftmals keine Beschwerden bereitet, die ihn an die regelmäßige Einnahme des Medikaments erinnern, ist es aus Gründen der Patienten-Compliance wünschenswert, eine Urapidil-Arzneiform zu haben, die es erlaubt, die gesamte Tagesdosis auf einmal einzunehmen. Urapidil wird, wie bei Antihypertonika üblich, oft zusammen mit einem Diuretikum verabreicht. Um die Nachtruhe des Patienten nicht durch die erhöhte Diurese zu beeinträchtigen, wird das Diuretikum üblicherweise nur morgens verabreicht. Falls eine Zubereitungsform für Urapidil zu Verfügung stünde, mit der eine gesamte Tagesdosis morgens verabreicht werden kann, könnte diese zusammen mit dem Diuretikum oder auch als feste Kombination mit dem Diuretikum eingenommen werden. Der Patient hätte dann nur einmal täglich morgens sein antihypertonisches Medikament einzunehmen. Diese vereinfachte Einnahme würde die Patienten-Compliance weiter erheblich fördern.

Aus der DE-C3-2336218 ist eine orale Depot-Arzneiform bekannt, bei der der Arzneistoff in Form sphäroider Teilchen vorliegt, die mit einer Dialysemembran überzogen sind, die aus einem unlöslichen Celluloseether und einer freie Carboxylgruppen aufweisenden organischen Verbindung besteht. Nach dem in der DE-C3-2336218 angegebenen Prinzip gelingt es, Urapidil enthaltende Retardzubereitungen herzustellen, die den Wirkstoff in vitro weitgehend pH-unabhängig über einen langen Zeitraum freigeben. Mit dem nach der Lehre dieser Schrift gefertigten Urapidil-Handelsprodukt lassen sich bei zweimaliger täglicher Verabreichung in vivo therapiegerechte Blutspiegelverläufe erreichen. Versuche der Anmelderin zeigen, daß es nach der Lehre der genannten Schrift möglich ist, Urapidil-Retardformulierungen zu bereiten, die in vitro ein Freisetzungsverhalten zeigen, die sie für eine täglich einmalige Verabreichung geeignet erscheinen lassen. Jedoch zeigen humankinetische Untersuchungen, daß die Bioverfügbarkeit solcher Retardformen auf unannehmbar kleine Werte zurückgeht.

Auch Versuche der Anmelderin mit anderen üblichen Retardierungsverfahren [Lehmann, Dreher, Pharm.Ind. 31, 319-322 und 409-412 (1969)] ergeben, daß es nicht möglich ist, Urapidil-Zubereitungsformen herzustellen, die bei täglich einmaliger Gabe in vivo eine annehmbare Bioverfügbarkeit aufweisen.

Aus der DE-A1-2831164 ist es bekannt, die Freisetzung eines basischen Wirkstoffes aus einer mit einer porösen semipermeablen Diffusionshülle versehenen Retard-Zubereitung durch die Gegenwart von sauren Stoffen, die mit unlöslichen filmbildenden Substanzen überzogen sind, zu verzögern und pH-unabhängig zu machen. Auch aus dieser Schrift ist nicht zu entnehmen, daß es möglich sein könnte, Zubereitungen für die täglich einmalige Verabreichung eines Wirkstoffs zu - schaffen.

Aus der bereits genannten DE-C3-2336218 sind Urapidil und Fumarsäure im Molverhältnis 1:1,11 enthaltende Pellets bekannt (Beispiel 1), die mit einer Dialysemembran überzogen sind.

In der EP-A1-0032562 wird eine speziell auf den Wirkstoff Dipyridamol abgestimmte Retardform angegeben. Da Dipyridamol oberhalb eines pH-Wertes von 4 praktisch wasserunlöslich ist, kann es nur im oberen Gastrointestinaltrakt gelöst und damit resorbiert werden. Diese Unlöslichkeit bei höherem pH-Wert wird durch den an sich bekannten Zusatz von Säuren, wie z.B. Weinsäure, ausgeglichen. Der Wirkstoff und die Säure werden mit einer Mem bran umgeben, die eine rasche Neutralisation der Säure verhindert und speziell an die Freigabecharakteristik für Dipyridamol angepaßt ist. Die aus dieser Schrift entnehmbare Lehre ist nicht auf Retardformen für Urapidil übertragbar, da Urapidil auch bei den höheren pH-Werten des Darmes eine ausreichende Löslichkeit besitzt. Hinweise für die Formulierung einer einmal täglichen Verabreichung sind der Schrift nicht zu entnehmen.

Aufgabe der vorliegenden Erfindung war es, eine technisch einfach herstellbare Zubereitungsform für den Wirkstoff Urapidil zur Verfügung zu stellen, mit der auch bei einmal täglicher Verabreichung eine annehmbare Bioverfügbarkeit des Wirkstoffs erreicht wird.

## Beschreibung der Erfindung

Überraschenderweise wurde nun festgestellt, daß diese Aufgabe gelöst werden kann, indem Urapidil zusammen mit pharmakologisch annehmbaren aliphatischen Dicarbonsäuren in einem Molverhältnis zwischen 0,05 und 0,7 in Pellets eingearbeitet wird und die Pellets mit einem magensaftresistenten Überzug versehen werden.

Gegenstand der Erfindung ist daher eine Retardpellets-Arzneiform enthaltend Urapidil und eine pharmakologisch annehmbare aliphatische Dicarbonsäure, die dadurch gekennzeichnet ist, daß das Molverhältnis Urapidil zu Dicarbonsäure zwischen 0,05 und 0,7 liegt und die Pellets mit einem magensaftresistenten Überzug versehen sind.

Die Dicarbonsäuren sollen nicht zu wasserunlöslich und nicht zu sehr wasserlöslich sein. Bevorzugt sind Dicarbonsäuren mit einer Löslichkeit von 0,004 bis 5 g, vorzugsweise 0,05 bis 3 g, pro 100 ml Wasser bei 20°C.

Besonders in Frage als Dicarbonsäuren kommen Fumarsäure und C6-C12-Dicarbonsäuren. Unter C6-C12-Dicarbonsäuren werden aliphatische Dicarbonsäuren mit sechs bis zwölf Kohlenstoffatomen verstanden. Die C6-C12-Dicarbonsäuren sind vorzugsweise gesättigt. Sie sind weiterhin vorzugsweise unverzweigt. α,ω-Dicarbonsäuren sind besonders bevorzugt. Besonders bevorzugt sind neben Fumarsäure Suberinsäure (C8) und Sebacinsäure (C10). Im Rahmen der vorliegenden Erfindung ist es auch möglich, Gemische der in Frage kommenden Säuren einzusetzen.

Das Molverhältnis zwischen Urapidil und der Dicarbonsäure wird auf einen Wert zwischen 0,05 und 0,7 eingestellt. Vorzugsweise liegt der Wert für das Molverhältnis zwischen 0,1 und 0,5. Bei Verwendung von Fumarsäure als Dicarbonsäure hat sich ein Molverhältnis von 0,1 bis 0,2 als günstig erwiesen.

Anstelle von Urapidil können erfindungsgemäß auch Salze von Urapidil mit den Dicarbonsäuren eingesetzt werden. Bei der Bestimmung des Molverhältnisses zwischen Urapidil und den Dicarbonsäuren ist der Säureanteil des Urapidilsalzes mit zu berücksichtigen. Beispielsweise ergibt sich im Sinne der vorliegenden Erfindung bei Verwendung von 1 Mol Urapidilsalz und zusätzlichen 2 Mol freier Säure ein Molverhältnis von 1 : (1 + 2) = 0,33. Vorzugsweise wird Urapidilfumarat mit überschüssiger Fumarsäure eingesetzt.

Als magensaftresistente Lacke kommen die zur Herstellung magensaftresistenter Überzüge üblichen Stoffe in Frage. Besonders geeignet für die Zwecke der vorliegenden Erfindung sind solche Lacke, die sich in einem pH-Bereich von 7,0 bis 7,5 lösen. Hier sind die polymeren Lacksubstanzen auf Acrylat-oder Methacrylatbasis zu nennen, insbesondere das im Handel als Eudragit®S erhältliche · anionische Polymerisat aus Methacrylsäure und Methacrylsäureestern.

Ein weiterer Gegenstand sind erfindungsgemäße Retardpellet-Arzneiformen, bei denen die verwendeten Dicarbonsäuren in Wasser von 20°C eine Löslichkeit von 0,004 bis 5 g, vorzugsweise 0,05 bis 3 g pro 100 ml Wasser, aufweisen.

Ein weiterer Gegenstand sind erfindungsgemäße Retardpellet-Arzneiformen, bei denen die Dicarbonsäuren aus der Gruppe Fumarsäure und gesättigte C6-C12-Dicarbonsäuren, insbesondere α,ω-C6-C12-Dicarbonsäuren, ausgewählt sind.

Ein weiterer Gegenstand sind erfindungsgemäße Retardpellet-Arzneiformen, bei denen Urapidil als Urapidilfumarat vorliegt und als Dicarbonsäure Fumarsäure verwendet wird, wobei das Molverhältnis von Urapidil einerseits zu Fumarsäure und Fumarsäureanion andererseits zwischen 0,1 und 0,2, vorzugsweise bei 0,15, liegt.

Ein weiterer Gegenstand sind erfindungsgemäße Retardpellet-Arzneiformen, bei denen sich der magensaftresistente Überzug bei einem pH-Wert von 7,0 bis 7,5 löst.

Ein weiterer Gegenstand sind. erfindungsgemäße Retardpellet-Arzneiformen, bei denen als magensaftresistenter Überzug polymere Lacksubstanzen auf Acrylat-oder Methacrylatbasis verwendet werden.

Ein weiterer Gegenstand sind erfindungsgemäße Retardpellet-Arzneiformen, bei denen als polymere Lacksubstanz ein anioniches Polymerisat aus Methacrylsäure und Methacrylsäureestern (Eudragit®S) verwendet wird.

Die Herstellung der Pellets erfolgt auf an sich bekannte Weise. Urapidil und/oder ein Salz von Urapidil mit der vorgesehenen Dicarbonsäure wird mit den üblichen Hilfsstoffen, wie z.B. mikrokristalliner Cellulose und einem Vinylpyrrolidon-Vinylacetat-Copolymerisat in einem Mischer gemischt und anschließend extrudiert. Die extrudierten Teilchen werden zu Pellets der gewünschten Korngröße abgerundet und getrocknet.

Das Auftragen des magensaftresistenten Lackes erfolgt auf an sich übliche Weise. Die Menge an magensaftresistentem Überzug beträgt etwa 8 bis 30 % des Gewichts der Pelletkerne abhängig von dem durchschnittlichen Durchmesser der Pellets. Dem Fachmann ist es geläufig, die relative Menge des Lacks bei Pellets mit größerem Durchmesser entsprechend zu verringern. Der Überzug kann neben magensaftresistentem Lack die für solche Überzüge üblichen Hilfsmittel, wie z.B. Weichmacher (z.B. Triacetin, Phthalate, Citronensäureester) und Stoffe zur Verringerung der Klebeneigung (z.B. Talkum, Magnesiumstearat) enthalten. Die Beschichtung wird vorteilhaft in einem Wirbelschichtsprühgranulator vorgenommen.

Ein weiterer Gegenstand der Erfindung ist ein Verfahren zur Herstellung einer Retardpellet-Arzneiform enthaltend Urapidil und eine pharmakologisch annehmbare aliphatische Dicarbonsäure, das dadurch gekennzeichnet ist, daß man Urapidil und Dicarbonsäure in einem Molverhältnis von 0,05 bis 0,7 in Pellets einarbeitet und die Pellets mit einem magensaftresistenten Überzug versieht.

Ein bevorzugter Gegenstand der Erfindung ist ein Verfahren, das dadurch gekennzeichnet ist, daß man Urapidil oder Urapidilfumarat und Fumarsäure in einem Molverhältnis Urapidil zu gesamter Fumarsäure von 0,1 bis 0,2 zusammen mit Hilfsstoffen extrudiert und zu Pellets einer Korngröße von 0,9 bis 1,4 mm abrundet und die Pellets mit einer Lösung eines anionischen Polymerisats aus Methacrylsäure und Methacrylsäureester beschichtet.

Die nachfolgenden Herstellungsbeispiele dienen zur Verdeutlichung der Erfindung.

## Herstellungsbeispiele

1. Für eine tägliche Einmaldosierung sind 120 mg Urapidil in der erfindungsgemäßen Form angebracht. Die nachfolgend angegebenen Mengen sind für die Füllung von 25.000 Kapseln berechnet.

Urapidilfumarat     4.500 g
Fumarsäure     6.000 g
Mikrokristalline Cellulose (Avicel® RC 581)     3.750 g
Vinylpyrrolidon-Vinylacetat-Copolymerisat (Kollidon® VA 64)     750 g
demineralisiertes Wasser     6.900 g

Urapidilfumarat, Fumarsäure, Avicel® RC 581 werden mit wäßriger Kollidon®-Lösung in einem Mischer befeuchtet. Die feuchte Mischung wird extrudiert und in einem Spheronizer zu Pellets der Korngröße von 0,9 bis 1,4 mm abgerundet. Nach dem Trocknen der Pellets werden diese mit einer Lösung aus

Anionisches Methacrylsäure/Methacrylsäureester-Polymerisat (Eudragit® S 100)     1.650 g
Citronensäureester (Citroflex® A 2)     165 g
Aceton     4.710 g
Isopropanol     18.450 g

in einem Wirbelschichtsprühgranulator beschichtet. Man erhält ca. 15 kg der erfindungsgemäßen Pellets.

Das Molverhältnis Urapidil zur Gesamtmenge an Fumarsäure beträgt in der vorstehenden erfindungsgemäßen Formulierung 0,148.

1 g Pellets enthalten ca. 200 mg Urapidil. Für eine Einmaldosierung werden 600 mg Pellets in eine Kapsel der Größe 00 abgefüllt.

2. Urapidil     4.500 g
Sebacinsäure     3.600 g
Mikrokristalline Cellulose (Avicel® RC 581)     1.400 g
Vinylpyrrolidon-Vinylacetat-Copolymerisat (Kollidon® VA 64)     300 g
demineralisiertes Wasser     4.000 g
Anionisches Methacrylsäure/Methacrylsäureester-Polymerisat (Eudragit® S 100)     800 g
Citronensäureester (Citroflex® A 2)     80 g
Aceton     2.000 g
Isopropanol     9.000 g

Die Verarbeitung erfolgt wie im Herstellungsbeispiel 1 angegeben.

Man erhält ca. 10 kg erfindungsgemäße Pellets, ausreichend für 35.000 Kapselfüllungen. Das Molverhältnis Urapidil zur Gesamtmenge an Sebacinsäure beträgt 0,68. 1 g Pellets enthalten 420 mg Urapidil. Für eine Einmaldosierung werden 285 mg Pellets in eine Kapsel der Größe 1 gefüllt.

3. Urapidil     4.000 g
Suberinsäure     4.000 g
Mikrokristalline Cellulose (Avicel® RC 581)     2.500 g
Vinylpyrrolidon-Vinylacetat-Copolymerisat (Kollidon® VA 64)     500 g
demineralisiertes Wasser     4.500 g
Anionisches Methacrylsäure/Methacrylsäureester-Polymerisat (Eudragit® S 100)     1.200 g
Citronensäureester (Citroflex® A 2)     120 g
Aceton     3.000 g
Isopropanol     13.500 g

Die Verarbeitung erfolgt wie im Herstellungsbeispiel 1 angegeben.

Man erhält ca. 11 kg erfindungsgemäße Pellets, ausreichend für 34,000 Kapselfüllungen. Das Molverhältnis Urapidil zur Gesamtmenge an Suberinsäure beträgt 0,45. 1 g Pellets enthalten 324 mg Urapidil. Für eine Einmaldosierung werden 308 mg Pellets in eine Kapsel der Größe 1 gefüllt.

Die so hergestellte Zubereitung setzt in vitro (USP XXI Paddle-Modell, 100 Umdrehungen in der Minute) den Wirkstoff im pH-Bereich von 1,0 bis 6,0 erwartungsgemäß praktisch nicht frei. Bei einem pH-Wert von 7,4 wird der Wirkstoff in diesem Modell innerhalb von 3 Stunden komplett freigesetzt.

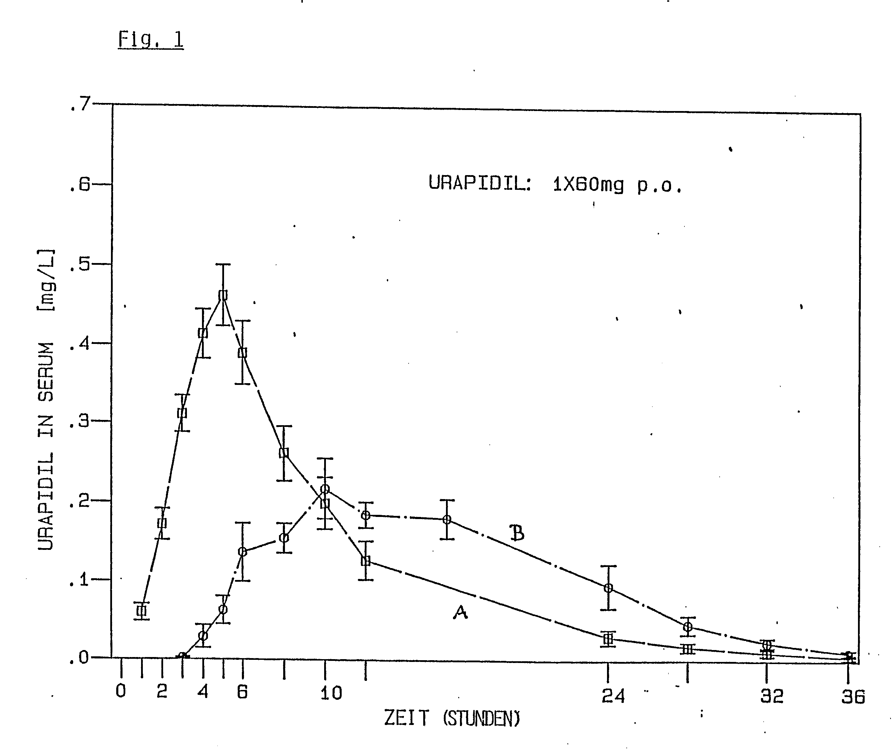

In Fig. 1 sind die Urapidil-Serumspiegelverläufe (mg/L) für eine erfindungsgemäße Zubereitung nach dem Herstellungsbeispiel 1 (Kurve B) und für die im Handel befindliche Urapidil-Retardzubereitung (Kurve A) aufgetragen. 6 (Kurve A) bzw. 12 (Kurve B) Probanden wurden zum Zeitpunkt 0 jeweils 60 mg Urapidil oral verabreicht. Wie sich aus den Kurven entnehmen läßt, liegt der Restblutspiegel 24 Stunden nach der Einnahme für die erfindungsgemäße Zubereitung rund 5mal so hoch wie für das Handelsprodukt und damit noch in nahezu idealer Weise im therapeutischen Bereich. Dieses überraschende Verhalten der erfindungsgemäßen Zubereitung ist aus dem in vitro beobachteten Freisetzungsverhalten nicht vorhersehbar. Besonders erstaunlich erscheint auch, daß trotz der in vitro beobachteten starken pH-Abhängigkeit der Freisetzung in vivo nur sehr geringe interindividuelle Streuungen der Serumspiegel auftreten.

## Ansprüche

1. Retardpellet-Arzneiform enthaltend Urapidil und eine pharmakologisch annehmbare aliphatische Dicarbonsäure, dadurch gekennzeichnet, daß das Molverhältnis Urapidil zu Dicarbonsäure zwischen 0,05 und 0,7 liegt und die Pellets mit einem magensaftresistenten Überzug versehen sind.

2. Arzneiform nach Anspruch 1, dadurch gekennzeichnet, daß die Carbonsäure in Wasser von 20°C eine Löslichkeit von 0,004 bis 5 g pro 100 ml aufweist.

3. Arzneiform nach Anspruch 1, dadurch gekennzeichnet, daß die Dicarbonsäuren aus der Gruppe Fumarsäure und gesättigte C6-C12-Dicarbonsäuren ausgewählt sind.

4. Arzneiform nach Anspruch 3, dadurch gekennzeichnet, daß als Dicarbonsäure Fumarsäure, Suberinsäure oder Sebacinsäure zugegen ist.

5. Arzneiform nach Anspruch 1, dadurch gekennzeichnet, daß Urapidil als Salz der Fumarsäure vorliegt und daß als Dicarbonsäure Fumarsäure verwendet wird, wobei das Molverhältnis von Urapidil einerseits zu Fumarsäure und Fumarsäureanion andererseits zwischen 0,1 und 0,2 liegt.

6. Arzneiform nach Anspruch 1, dadurch gekennzeichnet, daß als magensaftresistenter Überzug ein solcher verwendet wird, der sich in einem pH-Bereich von 7,0 bis 7,5 löst.

7. Arzneiform nach Anspruch 1, dadurch gekennzeichnet, daß als magensaftresistenter Überzug polymere Lacksubstanzen auf Acrylat- oder Methacrylatbasis verwendet werden.

8. Arzneiform nach Anspruch 7, dadurch gekennzeichnet, daß als polymere Lacksubstanz ein anionisches Polymerisat aus Methacrylsäure und Methacrylsäureestern (Eudragit®S) verwendet wird.

9. Verfahren zur Herstellung einer Retardpellet-Arzneiform enthaltend Urapidil und eine pharmakologisch annehmbare aliphatische Dicarbonsäure, dadurch gekennzeichnet, daß man Urapidil und Dicarbonsäure in einem Molverhältnis von 0,05 bis 0,7 in Pellets einarbeitet und die Pellets mit einem magensaftresistenten Überzug versieht.

10. Verfahren nach Anspruch 9, dadurch gekennzeichnet, daß man Urapidil oder Urapidilfumarat und Fumarsäure in einem Molverhältnis Urapidil zu gesamter Fumarsäure von 0,1 bis 0,2 zusammen mit Hilfsstoffen extrudiert und zu Pellets einer Korngröße von 0,9 bis 1,4 mm abrundet und die Pellets mit einer Lösung eines anionischen Polymerisats aus Methacrylsäure und Methacrylsäureester beschichtet.

Fig. 1

0 275 444

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | FR-A-2 237 620 (BYK GULDEN LOMBERG) * Seiten 18-21, Beispiele 1,2 * & DE-A-2 336 218 (Kat. D,Y) --- | 1-10 | A 61 K 31/505 A 61 K 9/52 |
| D,Y | EP-A-0 032 562 (Dr. KARL THOMAE GmbH) * Seite 17, Beispiel 1; Seite 21, Beispiel 6A * --- | 1-10 | |
| A | EP-A-0 122 077 (ELAN CORP.) * Seite 3, Zeile 16 - Seite 6, Zeile 8 * --- | | |
| T | CHEMICAL ABSTRACTS, Band 108, Nr. 5, 1. Februar 1988, Seite 11, Zusammenfassung Nr. 31231d, Columbus, Ohio, US; Y. KOIKE et al.: "Pharmacokinetics and pharmacodynamic effects of sustained release formulation of urapidil (BKU) in healthy subjects. Results of repeated oral administration", & RINSHO YAKURI 1987, 18(3), 579-88 * Zusammenfassung * ----- | | RECHERCHIERTE SACHGEBIETE (Int. Cl.4) A 61 K |

Der vorliegende Recherchenbericht wurde für alle Patentansprüche erstellt

| Recherchenort | Abschlußdatum der Recherche | Prüfer |
|---|---|---|
| DEN HAAG | 10-03-1988 | BENZ K.F. |